# EUROPÄISCHE PATENTSCHRIFT

(11) **EP 2 192 120 B1**
(45) Veröffentlichungstag und Bekanntmachung des Hinweises auf die Patenterteilung: **26.10.2011**
(21) Anmeldenummer: 09178428.0
(22) Anmeldetag: 08.04.2000
(51) Int. Cl.: C07D 475/04, A61K 31/519, A61P 39/02

(54) **Stabiles kristallines Calcium-Salz der 5-Methyl-(6S)-tetrahydrofolsäure**
Stable crystalline calcium salt of 5-methyl-(6S)-tetrahydrofolic acid
Sel cristallin stable de calcium de l'acide 5-méthyl-(6S)-tétrahydrofolique

(30) Priorität: 15.04.1999 CH 6951999
(43) Veröffentlichungstag der Anmeldung: 02.06.2010
(62) Teilanmeldung aus: 00107623.1
(73) Patentinhaber: Merck & Cie, 8200 Schaffhausen (CH)
(72) Erfinder: Müller, Hans Rudolf, 8207, Schaffhausen (CH); Egger, Thomas, 9545 Wängi (CH); Moser, Rudolf, 8200, Schaffhausen (CH)

(56) Entgegenhaltungen:
- EP-A- 0 455 013
- EP-A- 0 539 987
- EP-A2- 0 348 641
- DE-A1- 2 807 393

## Beschreibung

Die Erfindung betrifft das kristalline Calcium-Salz der N-[4-[[(2-amino-1,4,5,6,7,8-hexahydro-4-oxo-5-methyl-(6S)-pteridinyl)methyl]amino]benzoyl]-L-glutaminsäure (im folgenden 5-Methyl-(6S)-tetrahydrofolsäure Typ IV genannt), dessen Verwendung sowie ein Verfahren zu dessen Herstellung.

Verwendet werden Tetrahydrofolate vorwiegend als 5-Formyltetrahydrofolsäure und deren Salze (Leucovorin) oder 5-Methyltetrahydrofolsäure und deren Salze zur Behandlung von megaloblastischer Folsäure-Anämie, als Antidot zur Verstärkung der Verträglichkeit von Folsäure-Antagonisten, speziell von Aminopterin und Methotrexat in der Krebstherapie ("Antifolate rescue"), zur Verstärkung des therapeutischen Effektes von fluorierten Pyrimidinen und der Behandlung von Autoimmunkrankheiten wie Psoriasis und rheumatischer Arthritis, zur Verstärkung der Verträglichkeit von bestimmten Antiparasitika, etwa Trimethoprim-Sulfamethoxazol sowie zur Verminderung der Toxizität von Dideazatetrahydrofolaten in der Chemotherapie. 5-Methyltetrahydrofolsäure wird speziell eingesetzt als Arzneimittel und als Nahrungsmittelergänzungsstoff, Vitaminpräparat, zur Prävention von Neuralrohrdefekten, zur Behandlung von depressiven Erkrankungen und zur Beeinflussung des Homocysteinspiegels.

5-Methyltetrahydrofolsäure und deren Salze sind extrem instabil, wobei insbesondere die hohe Oxidationsempfindlichkeit auffällt [siehe dazu auch A.L. Fitzhugh, Pteridines 4(4), 187-191 (1993)] und daher schwierig in einer für einen pharmazeutischen Wirkstoff oder Nahrungsmittelergänzungsstoff akzeptablen Reinheit herzustellen.

Zur Überwindung der Instabilität der 5-Methyltetrahydrofolsäure wurden verschiedene Methoden wie ein möglichst vollständiger Sauerstoffausschluss oder die Zugabe von Oxidationsschutzmitteln wie Ascorbinsäure oder reduziertes L-Glutathion angewendet. Ein vollständiger Sauerstoffausschluss ist jedoch bei der Verwendung kaum, und wenn dann nur mit sehr grossem Aufwand zu realisieren, und die Zugabe von Oxidationsschutzmitteln ist ebenfalls nicht immer möglich. Bisher konnte demnach noch kein technisch gangbares Verfahren gefunden werden, das für die Herstellung von ausreichend stabilen Calcium-Salzen der 5-Methyltetrahydrofolsäure mit einer hohen Reinheit geeignet ist.

In der EP-A-0 455 013 ist die Herstellung des Calcium-Salzes der 5-Methyl-(6S)-tetrahydrofolsäure beschrieben. Ein Nacharbeiten der in Beispiel 3 beschriebenen Herstellung des Calcium-Salzes der 5-Methy)-(6S)-tetrahydrofolsäure aus dem Cyclohexylammonium-Salz der 5-Methyl-(6S)-tetrahydrofolsäure hat zu einem amorphen Produkt geführt.

In der EP-A-0 539 987 ist die Kristallisation des Calcium-Salzes der 5-Methyl-(6R,S)-tetrahydrofolsäure erwähnt. Das Nacharbeiten des angegebenen Beispiels zur Herstellung des Calcium-Salzes der 5-Methyl-(6R,S)-tetrahydrofolsäure aus dem Natrium-Salz der 5-Methyl-(6R,S)-tetrahydrofolsäure hat ein kristallines Produkt ergeben. Hingegen hat ein in einem Vergleichsbeispiel aus dem Natrium-Salz der 5-Methyl-(6S)-tetrahydrofolsäure unter gleichen Bedingungen hergestelltes Calcium-Salz der 5-Methyl-(6S)-tetrahydrofolsäure zu einem amorphen Produkt geführt.

Aus Beispiel 6 in der EP-A-0 773 221 ist es bekannt, dass das Calcium-Salz der (6S)-Tetrahydrofolsäure aus einer Lösung nach zwei Stunden Rühren bei 85°C auskristallisiert.

In DE OS 28 07 393 ist die Herstellung von 5-Methyl-(6R,S)-tetrahydrofolsäure und deren Salze beschrieben. Ein Nacharbeiten der in den Beispielen 1 und 7 beschriebenen Herstellung des Calcium-Salzes der 5-Methyl-(6R,S)-tetrahydrofolsäure hat zu amorphen Produkten geführt.

EP-A-0 682 026 beschreibt die fraktionierte Kristallisation von (6R,S)-Tetrahydrofolsäure bei bevorzugten pH-Werten für die Isolierung der (6S)-Tetrahydrofolsäure zwischen 3.5 und 6.5 bzw. zwischen 2 und 5.5 für die Isolierung der (6R)-Tetrahydrofolsäure.

In EP-A-0 535 710 wird die Herstellung des Calcium-Salzes der 10-Formyl-(6R)-tetrahydrofolsäure offenbart.

In EP-A-0 348 641 ist die Herstellung des Calcium-Salzes der 5-Methyl-(6S)-tetrahydrofolsäure beschrieben. Ein Nacharbeiten der in Beispiel 2.4 beschriebenen Herstellung des Calcium-Salzes der 5-Methyl-(6S)-tetrahydrofolsäure hat zu einem amorphen Produkt geführt.

EP-A-0 495 204 beansprucht ein Verfahren zur Herstellung von Sulfonsäure- und Schwefelsäure-Additionssalzen der Tetrahydrofolsäure.

EP-A-0 537 492 betrifft ein Verfahren zur Herstellung von Sulfonsäure- und Schwefelsäuresalzen der 5,10-Methylentetrahydrofolsäure.

Es wurde nun überraschend gefunden, dass sich das Calcium-Salz der 5-Methyl-(6S)-tetrahydrofolsäure mit einer hohen chemischen Reinheit und einer ausgezeichneten Stabilität erhalten lässt, indem das Salz aus einem polaren Medium nach einer Temperaturbehandlung der Lösung von über 60°C kristallisiert wird. Das so erhaltene hoch kristalline Calcium-Salz der 5-Methyl-(6S)-tetrahydrofolsäure ist bei Raumtemperatur praktisch unbeschränkt stabil. Es ist geeignet als Bestandteil oder als Ausgangsmaterial zur Herstellung von Arzneimittelformen oder Nahrungsmittelergänzungsstoffen.

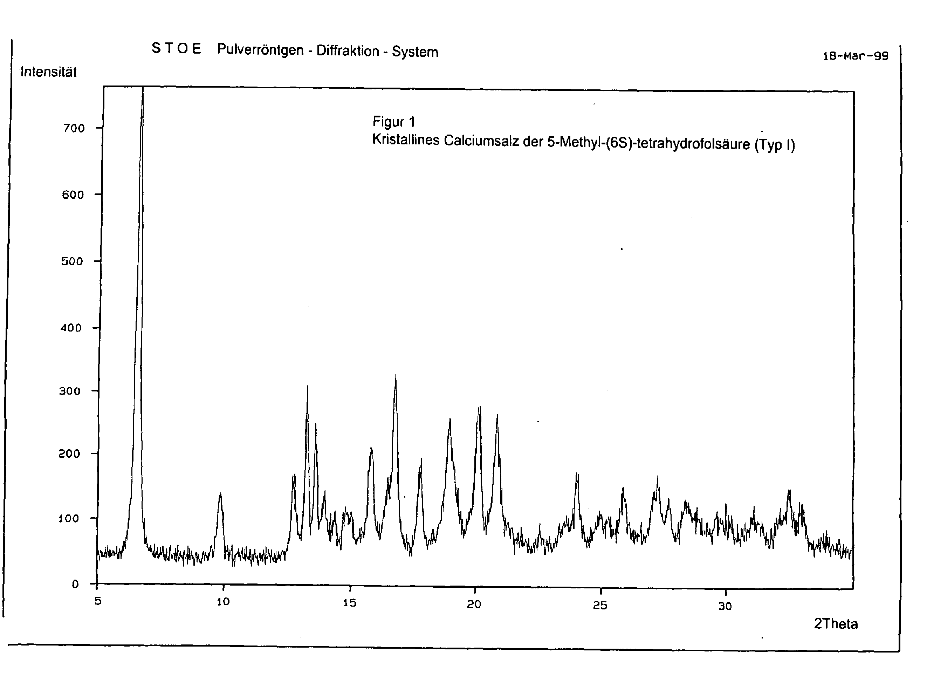

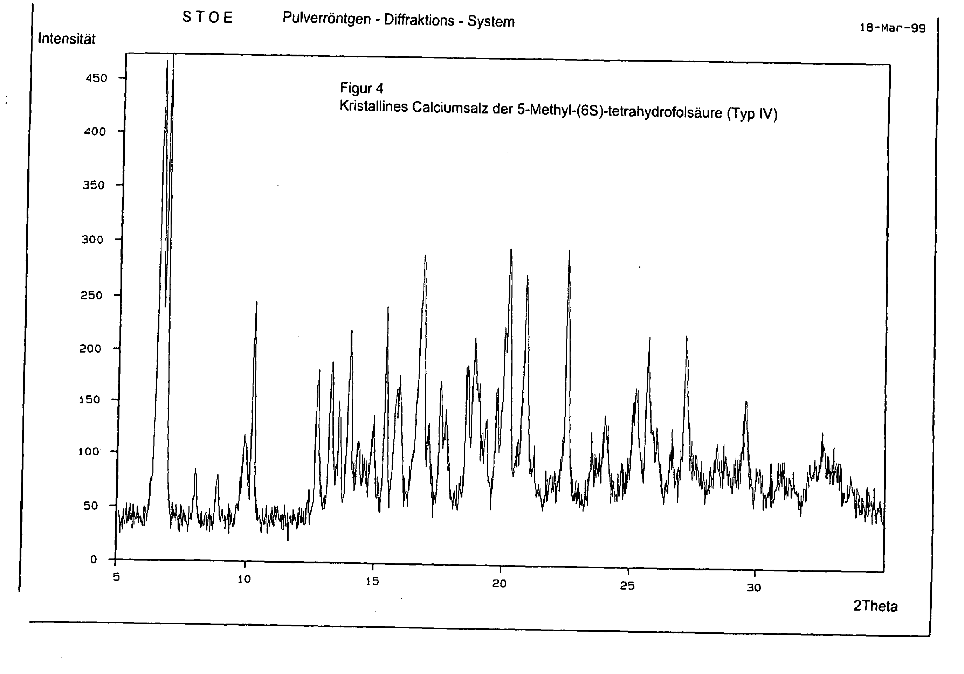

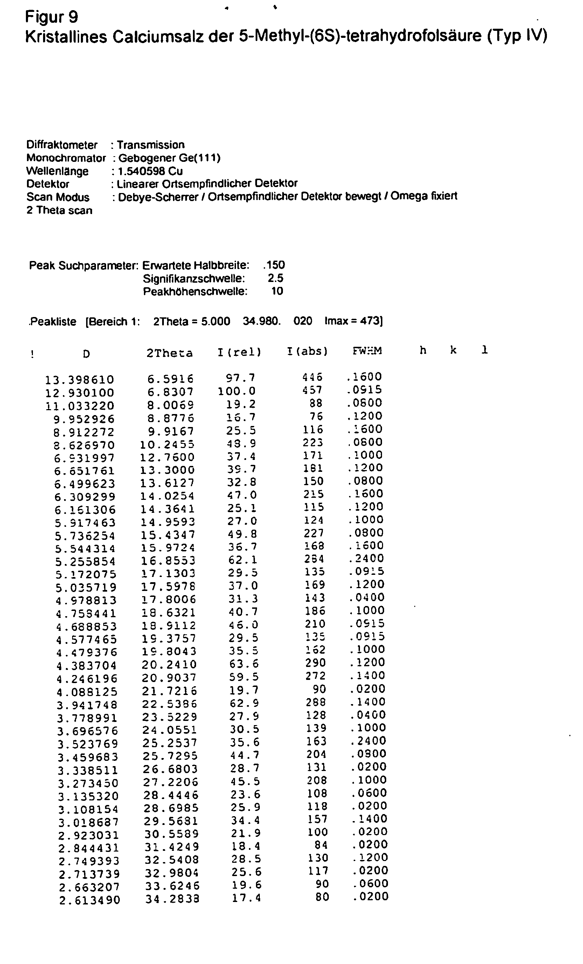

Gegenstand der Erfindung ist demnach ein kristallines Calcium-Salz der 5-Methyl-(6S)-tetrahydrofolsäure. Das kristalline Calcium-Salz der 5-Methyl-(6S)-tetrahydrofolsäure weist eine bisher nie erreichte Reinheit von > 98% zusammen mit einer bisher nie erreichten Stabilität von > 98% in Bezug auf den Ausgangswert nach 6 Monaten Lagerung unter Luft bei 25°C und 60% relativer Luftfeuchtigkeit auf. Das kristalline Calcium-Salz der 5-Methyl-(6S)-tetrahydrofolsäure liegt in der Kristallmodifikation Typ IV vor und zeigt bei Pulverröntgendiffraktions-Messungen scharfe Banden (siehe dazu Figur 4 und Figur 9). Ausgewählte 2 Theta-Werte für die Kristallmodifikation liegen 6.6, 15.9, 20.2 und 22.5 (Typ IV). Das kristalline Calcium-Salz der 5-Methyl-(6S)-tetrahydrofolsäure kann noch in weiteren Kristallmodifikationen (Typ I, Typ II und Typ III) vorliegen (siehe dazu Figuren 1, 2, 3, 6, 7 und 8). Ausgewählte 2 Theta-Werte für diese Kristallmodifikationen liegen bei 6.5, 13.3, 16.8 und 20.1 (Typ I); bzw. 5.3, 6.9, 18.7 und 21.1 (Typ II), bzw. 6.8, 10.2, 15.4 und 22.5 (Typ III). Das kristalline Calcium-Salz der 5-Methyl-(6S)-tetrahydrofolsäure weist einen Kristallwassergehalt von mindestens 1 Aequivalent Wasser auf 1 Aequivalent 5-Methyl-(6S)-tetrahydrofolsäure auf. So enthält die Modifikation Typ I typischerweise ≥ 3 Aequivalente Wasser, die Modifikation Typ II typischerweise ≤ 2 Aequivalente Wasser und die Modifikationen Typ III und Typ IV typischerweise ≤ 3 Aequivalente Wasser.

Ein weiterer Gegenstand der Erfindung ist das Verfahren zur Herstellung des hoch kristallinen Calcium-Salzes der 5-Methyl-(6S)-tetrahydrofolsäure Typ IV, welches dadurch gekennzeichnet ist, dass das Salz aus einem polaren Medium nach einer Temperaturbehandlung von über 60°C, speziell über 85°C wird.

Als polares Medium eignen sich vor allem Wasser oder ein Gemisch aus Wasser und einem mit Wasser mischbaren organischen Lösungsmittel wie wasserlöslichen Alkoholen, z. B. Methanol, Ethanol, n-Propanol, iso-Propanol, Ethylenglycol, einer niedrigen aliphatischen wasserlöslichen Carbonsäure, z. B. Ameisensäure, Essigsäure, Milchsäure oder wasserlöslichen Amiden, z. B. Formamid, Dimethylformamid, Dimethylacetamid, 1-Methylpyrrolidon, 2-Methylpyrrolidon, 2-Piperidinon. Es gibt keine besonderen Einschränkungen bezüglich der Art des eingesetzten Lösungsmittels und des Mischungsverhältnisses, das kristalline Calcium-Salz der 5-Methyl-(6S)-tetrahydrofolsäure eine tiefere Löslichkeiten als die entsprechende amorphe Form aufweist.

Die Kristallisation wird bevorzugt aus Lösungen durchgeführt. Die Kristallisation aus einer Suspension ist aber ebenfalls möglich.

Durch weitere Temperaturbehandlungen von über 60°C unter kontrollierter Luftfeuchtigkeit lassen sich die verschieden Kristallmodifikationen ineinander umwandeln. So kann Typ I, hergestellt durch eine Kristallisation aus einem polaren Medium nach einer Temperaturbehandlung von über 60°C, durch Trocknen im Vakuum bei 70°C in Typ II, durch eine Temperaturbehandlung von über 90°C in Typ III und durch eine Temperaturbehandlung von über 95°C in Typ IV umgewandelt werden. Typ II lässt sich durch Behandlung mit Wasser in einer Feuchtekammer bei 90°C wieder in Typ I zurückführen.

Die Kristallisation des Calcium-Salzes der 5-Methyl-(6S)-tetrahydrofolsäure erfolgt spontan oder durch Animpfen mit dem kristallinen Salz.

Als Ausgangsmaterial der Kristallisation eignet sich bevorzugt amorphe oder kristalline, reine 5-Methyl-(6S)-tetrahydrofolsäure.

Durch den Einsatz von amorpher oder teilkristalliner optisch reiner 5-Methyl-(6S)-tetrahydrofolsäure oder deren Calcium-Salz als Ausgangsmaterial für die Kristallisation erhält man durch das beschriebene Verfahren im Wesentlichen das kristalline Calcium-Salz der 5-Methyl-(6S)-tetrahydrofolsäure von bisher nie erreichter Reinheit zusammen mit einer bisher nie erreichten Stabilität.

Die Erfindung betrifft auch die Verwendung des hoch kristallinen Calcium-Salzes der 5-Methyl-(6S)-tetrahydrofolsäure Typ IV als Bestandteil zur Herstellung von Arzneimitteln oder Nahrungsmittelergänzungsstoffen oder zur Herstellung anderer Tetrahydrofolsäure-Derivate, da das kristalline Calcium-Salz der 5-Methyl-(6S)-tetrahydrofolsäure aufgrund seiner ausgezeichneten Stabilität in fester Form eine zeitlich praktisch unbeschränkt gleichbleibende sehr gute Qualität behält. Ebenso betrifft die Erfindung auch Zubereitungen enthaltend hoch kristalline Calcium-Salz der 5-Methyl-(6S)-tetrahydrofolsäure Typ IV. Die Herstellung der Zubereitungen erfolgt nach bekannten Verfahren. Die Anwendung erfolgt analog zur Anwendung von bekannten Substanzen aus dem Gebiet der Tetrahydrofolate wie z. B. 5-Formyltetrahydrofolsäure (Leucovorin).

Die vorliegende Beschreibung ermöglicht es dem Fachmann die Erfindung umfassend anzuwenden. Die folgenden Beispiele sind deshalb nur zur Veranschaulichung möglicher Varianten und daher in keiner Art und Weise einschränkend zu verstehen.

Alle in den folgenden Beispielen erwähnten Temperaturen sind in Grad Celsius angegeben. Wo nicht anders bezeichnet sind Gehaltsangaben als Gewichts-% aufgeführt.

### Beispiele zur Illustrierung der Erfindung

Der in den Beispielen angegebene Gehalt an 5-Methyltetrahydrofolsäure-Salz wurde jeweils mit HPLC bestimmt und in %-Fläche angegeben, der Wassergehalt wurde über eine Karl-Fischer-Methode bestimmt.

### Beispiel 1 [Stabilitäten]

Zur Stabilitätsbestimmung der kristallinen 5-Methyltetrahydrofolsäure-Salze wurden die Substanzen zusammen mit Vergleichsmustern bei 25°C und 60% relative Feuchte unter Luft gelagert. In periodischen Abständen wurde der verbleibende Gehalt an 5-Methyltetrahydrofolsäure-Salz gemessen und im Vergleich zum Ausgangswert angegeben.

| | **Belastungszeit in Monaten** | | | | | |
|---|---|---|---|---|---|---|
| | **0** | **3** | **6** | **12** | **18** | **88** |
| kristallines Calcium-Salz der 5-Methyl-(6S)-tetrahydrofolsäure | 100% | 98.6% | 98.7% | 99.1% | 99.0% | 97.8% |
| amorphes Calcium Salz der 5-Methyl-(6S)-tetrahydrofolsäure | 100% | | | 84.2% | | |

Kristalline 5-Methyltetrahydrofolsäure-Salze sind auch nach längerer Belastungszeit noch sehr hell. Im Gegensatz dazu verfärben sich die amorphen Proben rasch sehr stark.

### Beispiel 2 [Pulverröntgendiagramme]

Zur Charakterisierung der strukturellen Eigenschaften (Kristallmodifikationen) der kristallinen 5-Methyltetrahydrofolsäure-Salze wurden von diesen Substanzen Pulverröntgendiagramme (Beugungsspektren) aufgenommen.

Kristalline 5-Methyltetrahydrofolsäure-Salze ergeben gut aufgelöste Spektren mit scharfen Banden und niedrigem Untergrund. Die Spektren weisen auf hohe kristalline Anteile hin.

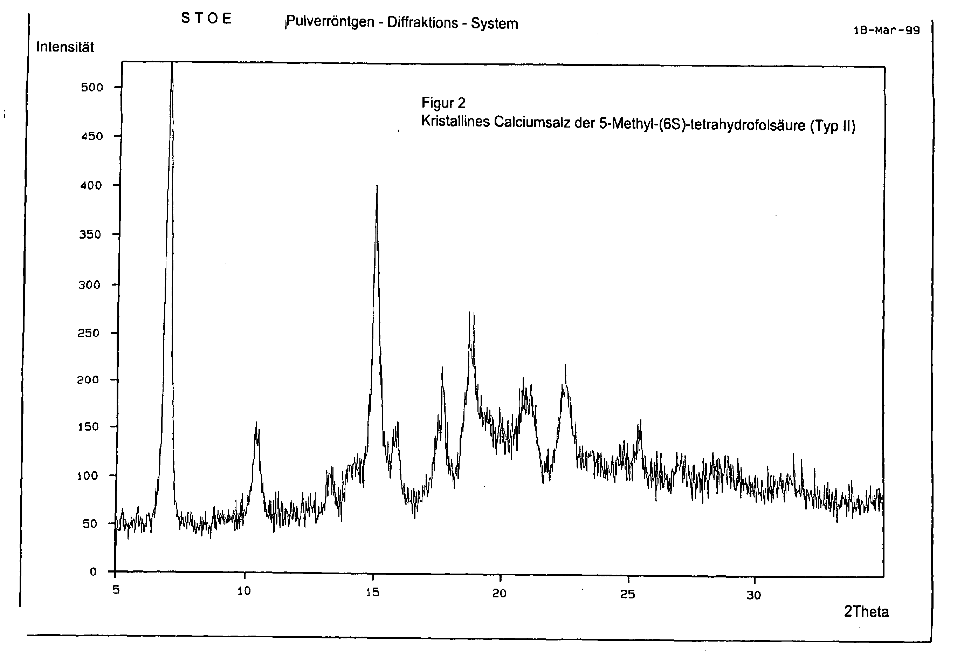

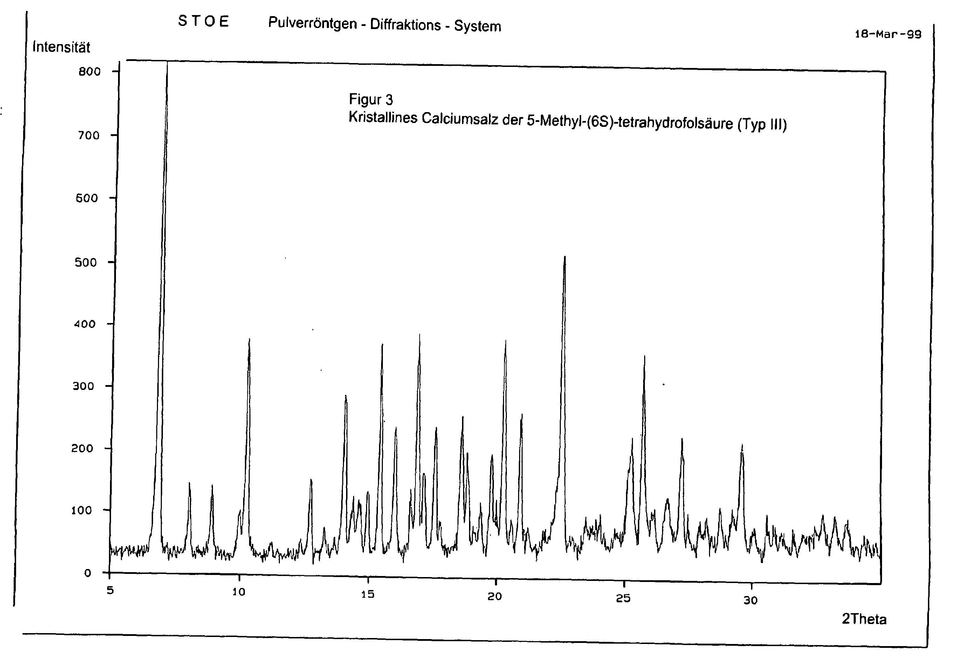

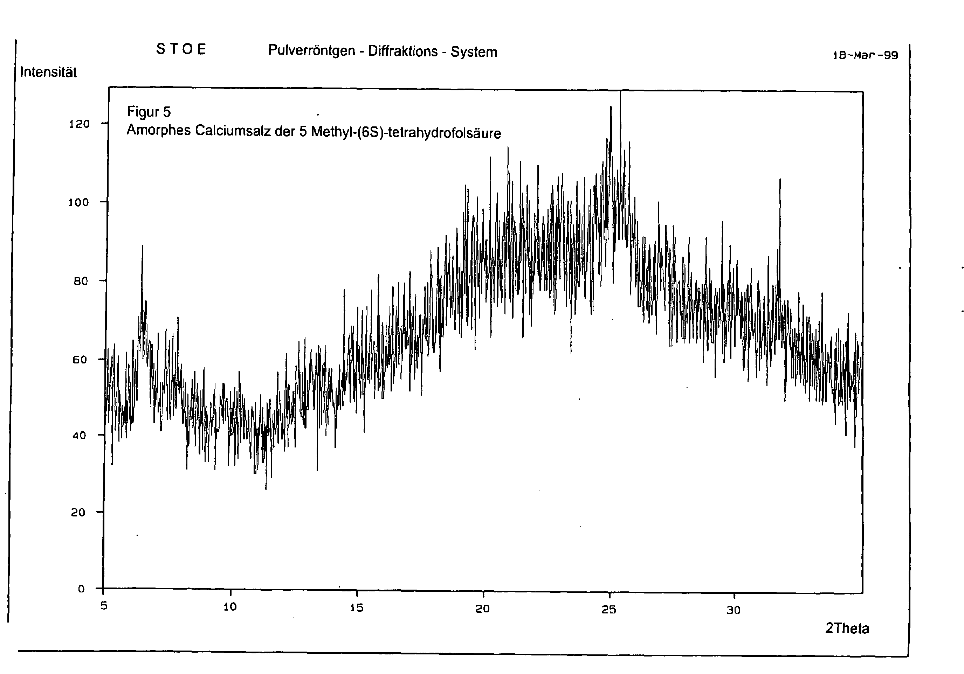

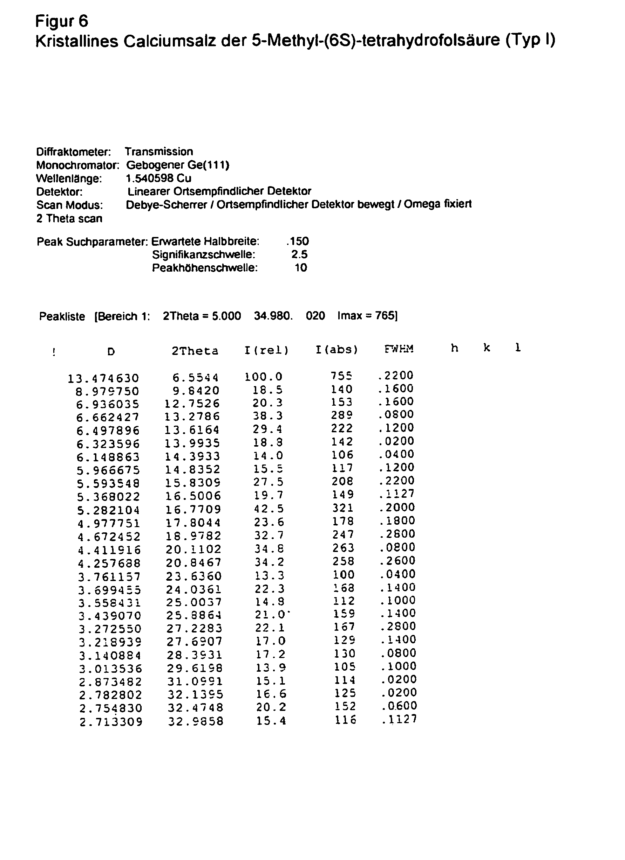

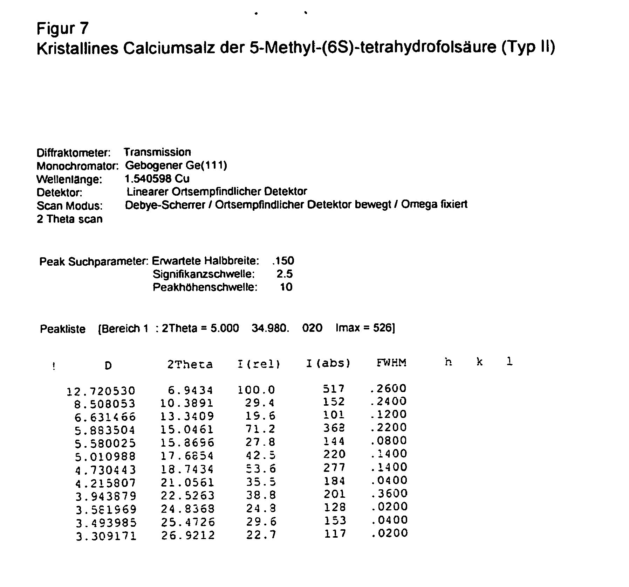

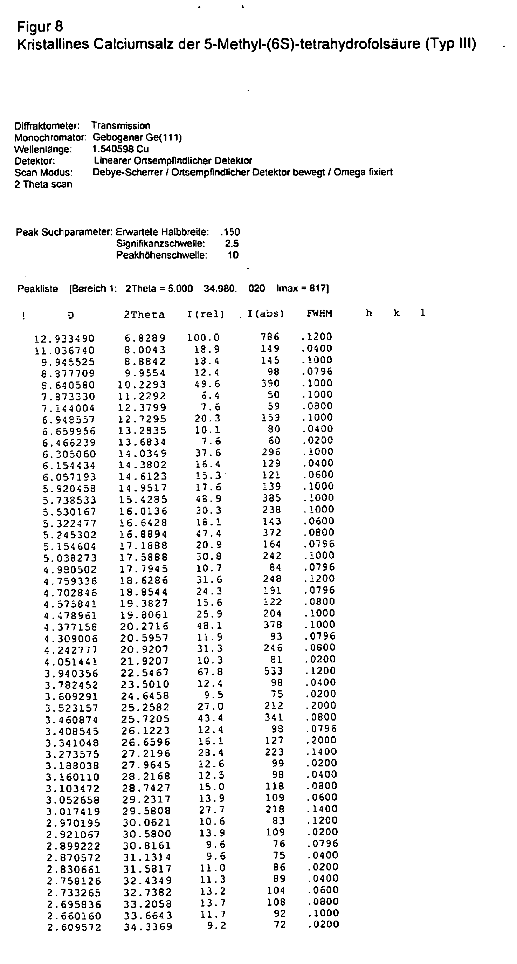

Beispiele von Spektren sind in Figur 1 (Typ I), Figur 2 (Typ II), Figur 3 (Typ III) und Figur 4 (Typ IV), bzw. in Figur 6 (Typ I), Figur 7 (Typ II), Figur 8 (Typ III) und Figur 9 (Typ IV) ersichtlich. Zum Vergleich wurde von einer amorphen Probe unter analogen Bedingungen ebenfalls ein Spektrum aufgenommen und als Figur 5 (amorph) aufgeführt.

Im Folgenden sind ausgewählte 2 Theta-Werte für die verschiedenen Kristallmodifikationen des kristallinen 5-Methyl-(6S)-tetrahydrofolsäure-Calcium-Salzes aufgelistet:

| **Typ** | **ausgewählte 2 Theta-Werte** |
|---|---|
| Typ I | 6.5, 13.3, 16.8 und 20.1 |
| Typ II | 5.3, 6.9, 18.7 und 21.1 |
| Typ III | 6.8, 10.2, 15.4 und 22.5 |
| Typ IV | 6.6, 15.9, 20.2 und 22.5 |

### Beispiel 3 [Löslichkeiten]

Die Löslichkeit von kristallinem 5-Methyl-(6S)-tetrahydrofolsäure-Calcium-Salz ist in der folgenden Tabelle aufgezeichnet:

| **Typ** | **Löslichkeit bei 20°C in** | |
|---|---|---|
| | **0.9% NaCl** | **Wasser** |
| Typ I | 1.6% | 1.1% |
| Typ II | 5.8% | 3.8% |
| Typ III | 1.5% | 1.0% |

### Beispiel 4 [amorphes 5-Methyl-(6S)-tetrahydrofolsäure-Calcium-Salz]

7.5 g 5-Methy)-(6S)-tetrahydrofolsäure werden unter Einleitung von N₂ bei Raumtemperatur in 75 ml Wasser eingetragen und mit Natronlauge 30% auf pH 12 eingestellt. Die so erhaltene klare Lösung wird mit Salzsäure 37% auf pH 7.5 eingestellt und mit einer Lösung von 7.15 g Calciumchlorid 6H₂O in 11.7 ml Wasser versetzt. Die entstandene weisse Suspension wird nach 5 Stunden ausrühren bei Raumtemperatur genutscht, mit Wasser überwaschen und bei 45°C im Vakuum getrocknet.

Man erhält 5.8 g weisses amorphes 5-Methyl-(6S)-tetrahydrofolsäure-Calcium-Salz mit einem Gehalt von 98.0% und einem 6S-Anteil von 99.6%.

Auch nach der Behandlung dieser Substanz bei 60°C in der Feuchtekammer können weder im Polarisationsmikroskop noch in einer Röntgendiffraktionsmessung kristalline Anteile bestimmt werden.

### Beispiel 5 [Typ I]

130 kg Wasser werden vorgelegt und 12.8 kg 5-Methyl-(6S)-tetrahydrofolsäure eingetragen. Mit ca. 9.1 kg NaOH 30% wird der pH-Wert auf 11.6 und dann mit ca. 1.9 kg Salzsäure 37% auf 7.6 eingestellt. Zur klaren Lösung wird eine Suspension enthaltend 0.3 kg Kohle und 0.3 kg Cellflock zugegeben, filtriert und mit 13 I Wasser überwaschen. Das Filtrat wird mit einer Lösung enthaltend 8.3 kg Calciumchlorid-2H₂O versetzt, auf 90°C erhitzt und 30 Minuten gerührt. Das Produkt wird heiss abfiltriert und mit 2 x 20 kg Wasser überwaschen. Das so erhaltene feuchte Rohprodukt wird in 115 I Wasser aufgeschlämmt, auf 90°C erhitzt, sofort heiss abfiltriert, mit 2 x 20 kg Wasser überwaschen und bei 40°C im Vakuum getrocknet.

Man erhält 11.6 kg weisses kristallines 5-Methyl-(6S)-tetrahydrofolsäure-Calcium-Salz (Typ I) mit einer Reinheit von 99.0% und einem Wassergehalt von 14.5%.

### Beispiel 6 [Typ I]

1600 ml Wasser werden vorgelegt und 194 g 5-Methyl-(6S)-tetrahydrofolsäure eingetragen. Mit ca. 80 ml NaOH 30% wird der pH-Wert auf 7.0 eingestellt. Zur klaren Lösung wird eine Suspension enthaltend 20 g Kohle und 20 g Cellflock in 190 ml Wasser zugegeben, filtriert und mit Wasser überwaschen. Das Filtrat wird mit einer 950 ml Calciumchlorid-Lösung 5.5 M versetzt, auf 90°C erhitzt und 60 Minuten gerührt. Das Produkt wird heiss abfiltriert und mit Wasser überwaschen und bei 45°C im Vakuum getrocknet.

Man erhält 156.2 g weisses kristallines 5-Methyl-(6S)-tetrahydrofolsäure-Calcium-Salz (Typ I) mit einer Reinheit von 99.7% und einem 6S-Anteil von 99.9%.

### Beispiel 7 [Typ I und Umwandlung in Typ II]

554 g Wasser werden vorgelegt und 53.1 g 5-Methyl-(6S)-tetrahydrofolsäure eingetragen. Mit NaOH 30% wird der pH-Wert auf 7.5 eingestellt. Zu klaren Lösung werden 1.3 g Kohle, 1.3 g Cellflock und 19.5 g Wasser zugegeben. Die Suspension wird filtriert und mit 55 ml Wasser überwaschen. Das Filtrat wird mit einer Lösung von 52.0 g Calciumchlorid·6H₂O in 84.6 g Wasser versetzt, auf 90°C erhitzt und mit 100 mg kristallinem 5-Methyltetrahydrofolsäure-Calcium-Salz angeimpft. Nach erfolgter Kristallisation wird das Produkt bei 90°C heiss abfiltriert und mit 2 x 103 g Wasser überwaschen. Das so erhaltene feuchte Rohprodukt wird in 480 ml Wasser aufgeschlämmt, auf 90°C erhitzt, sofort heiss abfiltriert, analog oben überwaschen und bei 45°C im Vakuum getrocknet.

Man erhält 47.5 g weisses kristallines 5-Methyl-(6S)-tetrahydrofolsäure-Calcium-Salz (Typ I) mit einer Reinheit von 98.8% und einem Wassergehalt von 12.2%.

Durch Trocknen bei 70°C im Vakuum während 30 Minuten kann diese Modifikation Typ I in die Modifikation Typ II mit einem Wassergehalt von 5.0% umgewandelt werden.

### Beispiel 8 [Typ III]

15.8 g 5-Methyl-(6S)-tetrahydrofolsäure-Calcium-Salz werden in 140 ml Wasser unter Einleitung von N₂ auf 95°C erhitzt, nach 30 Minuten bei 95°C wird die weisse Suspension heiss genutscht, mit Wasser überwaschen und bei 35°C im Vakuum getrocknet.

Man erhält 14.0 g weisses kristallines 5-Methyl-(6S)-tetrahydrofolsäure-Calcium-Salz (Typ III) mit einem Gehalt von 98.9% und einem 6S-Anteil von 99.9%.

### Beispiel 9 [Typ IV]

20.0 g 5-Methyl-(6S)-tetrahydrofolsäure-Calcium-Salz werden in 180 ml Wasser unter Einleitung von N₂ auf 100°C erhitzt, nach 30 Minuten bei 100°C wird die weisse Suspension heiss genutscht, mit Wasser überwaschen und bei 25°C im Vakuum getrocknet.

Man erhält 16.9 g weisses kristallines 5-Methyl-(6S)-tetrahydrofolsäure-Calcium-Salz (Typ IV) mit einem Gehalt von 98.3% und einem Wassergehalt von 9.9%.

Durch Trocknen bei 65°C im Vakuum kann der Wassergehalt dieses Produktes auf 5.5% reduziert werden, ohne dass dabei eine andere Kristallmodifikation erhalten wird.

## Patentansprüche

1. Kristallines Calcium-Salz der 5-Methyl-(6S)-tetrahydrofolsäure mit 2 Theta-Werten von 6.6, 15.9, 20.2 und 22.5.

2. Verfahren zur Herstellung des kristallinen Salzes gemäss Anspruch 1, **dadurch gekennzeichnet, dass** ein kristallines Salz der 5-Methyt-(6S)-tetrahydrofolsäure aus einem polaren Medium nach einer Temperaturbehandlung von über 60°C kristallisiert wird und durch weitere Temperaturbehandlung von über 60°C unter kontrollierter Luftfeuchtigkeit in das kristalline Salz gemäss Anspruch 1 umgewandelt wird.

3. Verfahren zur Herstellung des kristallinen Salzes gemäss Anspruch 1, **dadurch gekennzeichnet, dass** ein kristallines Salz der 5-Methyl-(6S)-tetrahydrofolsäure aus einem polaren Medium nach einer Temperaturbehandlung von über 85°C kristallisiert wird und durch weitere Temperaturbehandlung von über 60°C unter kontrollierter Luftfeuchtigkeit in das kristalline Salz gemäss Anspruch 1 umgewandelt wird.

4. Verfahren nach Anspruch 2 oder 3, **dadurch gekennzeichnet, dass** die Kristallisation aus einer Lösung durchgeführt wird.

5. Verfahren nach Anspruch 2 oder 3, **dadurch gekennzeichnet, dass** die Kristallisation aus einer Suspension durchgeführt wird.

6. Verfahren nach Anspruch 4 oder 5, **dadurch gekennzeichnet, dass** die Kristallisation aus Wasser oder einem Gemisch aus Wasser und einem mit Wasser mischbaren organischen Lösungsmittel durchgeführt wird.

7. Verwendung von kristallinen Salzen gemäss Anspruch 1 als Bestandteil zur Herstellung von Arzneimitteln oder als Nahrungsmittelergänzungsstoff.

8. Zubereitungen enthaltend kristalline Salze gemäss Anspruch 1.

## Claims

1. Crystalline calcium salt of 5-methyl-(6S)-tetrahydrofolic acid with 2 theta values of 6.6, 15.9, 20.2 and 22.5.

2. Method for producing crystalline salt according to claim 1, **characterised in that** a crystalline salt of 5-methyl-(6S)-tetrahydrofolic acid is crystallised from a polar medium after thermal treatment at over 60°C and converted into crystalline salt according to claim 1 by further thermal treatment at over 60°C with controlled air moisture.

3. Method for producing crystalline salt according to claim 1, **characterised in that** a crystalline salt of 5-methyl-(6S)-tetrahydrofolic acid is crystallised from a polar medium after thermal treatments at over 85°C and is converted into crystalline salt according to claim 1 by further thermal treatment at over 60°C with controlled air moisture.

4. Method according to claim 2 or 3, **characterised in that** crystallisation is carried out from a solution.

5. Method according to claim 2 or 3, **characterised in that** crystallisation is carried out from a suspension.

6. Method according to claims 4 or 5, **characterised in that** crystallisation is carried out from water or a mixture of water and an organic solvent, which may be mixed with water.

7. Use of crystalline salts according to claim 1 as a component for producing drugs or as a food supplement.

8. Preparations containing crystalline salts according to claim 1.

## Revendications

1. Sel de calcium cristallin de l'acide 5-méthyl-(6S)-tétrahydrofolique présentant les valeurs 2 thêta de 6.6, 15.9, 20.2 et 22.5.

2. Procédé de production du sel cristallin selon la revendication 1, **caractérisé en ce qu'**un sel cristallin de l'acide 5-méthyl-(6S)-tétrahydrofolique est cristallisé à partir d'un milieu polaire après un traitement thermique supérieur a 60°C et est converti par d'autres traitements thermiques supérieur à 60°C dans des conditions d'humidité contrôlées, en sel cristallin selon la revendication 1.

3. Procédé de production du sel cristallin selon la revendication 1, **caractérisé en ce qu'**un sel cristallin de l'acide 5-méthyl-(6S)-tétrahydrofolique est cristallisé à partir d'un milieu polaire après un traitement thermique supérieur a 85°C et est converti par d'autres traitements thermiques supérieur à 60°C dans des conditions d'humidité contrôlée, en sel cristallin selon la revendication 1.

4. Procédé selon la revendication 2 ou 3, **caractérisé en ce que** la cristallisation est réalisée à partir d'une solution.

5. Procédé selon la revendication 2 ou 3, **caractérisé en ce que** la cristallisation est réalisée à partir d'une suspension.

6. Procédé selon la revendication 4 ou 5, **caractérisé en ce que** la cristallisation est réalisée à partir d'eau ou d'un mélange d'eau et d'un solvant organique miscible avec l'eau.

7. Utilisation de sels cristallins selon la revendication 1, comme composant pour la production de médicaments ou d'additifs alimentaires.

8. Préparations contenant des sels cristallins selon la revendication 1.
